# EUROPEAN PATENT APPLICATION

(11) **EP 1 162 266 A2**
(43) Date of publication of application: **12.12.2001**
(21) Application number: 01202009.5
(22) Date of filing: 28.05.2001
(51) Int. Cl.: C12N 15/31, C12N 15/81

(54) **DNA fragments of the methylotrophic pichia pastoris yeast ICL gene**

(30) Priority: 26.05.2000 CU 12200
(71) Applicant: CENTRO DE INGENIERIA GENETICA Y BIOTECNOLOGIA, Ciudad Habana 12100 (CU)
(72) Inventor: Menéndez Diaz, Javier, Playa, Ciudad de la Habana, 10600 (CU); Valdés Prado, Iris, Calle Santa Felicia No. 427, Ciudad de la Habana, 10700 (CU); Cabrera Leòn, Nelson, Playa, Ciudad de la Habana, 10600 (CU)
(74) Representative: Prins, Adrianus Willem

(57) **Abstract**

This invention relates to the field of recombinant DNA technology. The present invention relates to the isolation of a DNA regulatory region, able to lead the expression of heterologous proteins in *Pichia pastoris*.
In one of its aspects the invention is related to the isolation of a DNA fragment that contains the isocitrate lyase encoding gene (referred here as *ICL*) from the yeast *Pichia pastoris*. In another aspects, this invention describes the use of a fragment derived from the described gene, which lead the expression of a foreign gene, when both are incorporated in a DNA vector and introduced in the host yeast *Pichia pastoris.* In this way the protein of interest is efficiently produced using this DNA fragment.

In the yeast *Pichia pastoris* the expression of this gene is regulated in response to environmental conditions, such as the growth medium composition. The *ICL* expression is repressed when a carbon source like glucose is used, and induced when the carbon source present in the medium is ethanol or when the glucose is absent.
An additional aspect, we have also isolated a DNA fragment consisting essentially of the *Pichia pastoris ICL* 3' transcription termination sequence.

SÉQUENSE LISTING

<110> CENTRO DE INGENIERIA GENETICA Y BIOTECNOLOGIA

<120> DNA fragments of the methylotrophic Pichia pastoris yeats ICL gene.

<130> Sequences Listing

<140> 2000/122
<141> 2000-05-26

<160> 10

<170> PatentIn Ver. 2.1

<210> 1
<211> 684
<212> DNA
<213> Pichia pastoris

<220>
<221> promoter
<222> (1)..(684)
<223> Sequence that contains the 5' regulatory region of ICL gene.

<400> 1
gaattcggac aaatgtgctg ttccggtagc ttgtaggaag cggcatccgt agggcaatat 60
acgactatag cttctaaagc gtagtacaat gaaatgttcg aaggaacaan aaacggattt 120
gtttttcgta ggctcaaccn gttgaggtgt aactctttag ngaaagggta agattgattg 180
ttcgaagtag gggcctcaaa gggaaagaga aaaaaaaaaa tacaccgaag agttacgtaa 240
gcatatattt tttacgtaaa gcatgattga atttcagcag tattgtttaa caaggctgat 300
gtcgtgtgcc aatcaaaaca aaagagattc gcataatgcc ataattgggg tgtgtgggng 360
nccccnaaaa cgtctttctc atcatcatct gcaaccccca tcgaacctca ttaaatcaca 420
tgacttgtgc gatcctcggt caactcgttc cgtgcaccca ttccaccccg ggctgaccaa 480
cgcaaggttc tccgagagtc cgctacccca gatttatatc agcaaccagt cacctttttc 540
cgggcacgac tctatatgcc ctggaaaacc ggagacgatg agcctgacta taaaaggtga 600
cagaaccccc aactctggtt aatctcttca acaaatactt tattttcttt caattcaaag 660
aacacagtat caagtatatc aaga 684

<210> 2
<211> 360
<212> DNA
<213> Pichia pastoris

<220>
<221> terminator
<222> (1)..(360)
<223> Sequence that contains the 3'non-translated region of ICL gene.

<400> 2
gtaataggag ttcctaagta gttaagataa ttgacttgag gtatttatag atttgtgtgt 60
aggtaatatc tatggtcgtc cattcttacc ttggtggggt gacggggcgg tgaataaatc 120
agttgcgatg aaagacttta caaccttgtc accagagggt gcggtctact gattactaca 180
aacgacttgg ataaaatttt caattcaaaa tcaatataaa aaaaaaaact taacatcact 240
gatgtttcac taaactcttt aaacgctcaa cctcagcttc caactcgctc ttgcaaatca 300
gtaactcctc aactttgtct tccagttgac tcattctctt catcttctta gccctggaac 360

<210> 3
<211> 21

<212> DNA
<213> Artificial sequence

<220>
<223> Description of the artificial sequence: oligonucleotide

<400> 3
tchggwtggc artgytchtc h                                                21


<210> 4
<211> 21
<212> DNA
<213> Artificial sequence

<220>
<223> Description of the artificial sequence: oligonucleotide


<400> 4
gtncahgarg aycarttyaa r                                                21


<210> 5
<211> 27
<212> DNA
<213> Artificial sequence

<220>
<223> Description of the artificial sequence: oligonucleotide

<400> 5
ctgcaggaat taattcgcct tagacat                                          27


<210> 6
<211> 26
<212> DNA
<213> Artificial sequence

<220>
<223> Description of the artificial sequence: oligonucleotide

<400> 6
aagcttgcgt taacgaatct agaact                                           26


<210> 7
<211> 23
<212> DNA
<213> Artificial sequence

<220>
<223> Description of the artificial sequence: oligonucleotide

<400> 7
cctcgagctt gtaggaattc gca                                              23


<210> 8
<211> 24

<212> DNA
<213> Artificial sequence

<220>
<223> Description of the artificial sequence: oligonucleotide

<400> 8
aaggtgctag cattcttgat atac                                              24


<210> 9
<211> 24
<212> DNA
<213> Artificial sequence

<220>
<223> Description of the artificial sequence: oligonucleotide

<400> 9
atgctagcgc aagctttcct tttc                                              24


<210> 10
<211> 24
<212> DNA
<213> Artificial sequence

<220>
<223> Description of the artificial sequence: oligonucleotide

<400> 10
agctcgcgat cagctaatct gcca                                              24

## Description

The development of the recombinant DNA technology has permitted the use of several microorganisms as host for the expression of heterologous proteins with pharmaceutical and industrial application. The yeast *Saccharomyces cerevisiae* is the most eukaryotic microorganisms frequently used in the current biotechnology. However, in the last decade, yeasts from the genus *Pichia*, *Hansenula, Candida* and *Kluyveromyces*, have been used efficiently as expression systems (P.E. Sudbery, 1994, Yeast 10: 1707-1726).

In this sense, could be of particular interest for these microorganisms, to have 5' regulatory regions (referred here like promoters) which provide for high levels of DNA transcription to RNA and are responsive to exogenous environmental stimulus. This type of promoter regions permit to split the growth phase from the production phase of the protein, avoiding the selection non-expressing strains.

In the last few years the *P. pastoris* expression system has rapidly achieved much wider use, thanks to increasing awareness of the earlier successes and a variety of vectors that allow the selection of transformants in the host strain (Cregg *et al.*, 1993, Bio/ Technology 11: 905-910). Most *Pichia* expression vectors are very similar and are based in the use of the promoter from the alcohol oxidase gene (*AOX1*). Between the advantages that have this promoter are the use of a cheaper carbon source, such as methanol for induces the expression of the recombinants proteins. Also this expression system is highly regulated favoring its use in expression of products that could be toxic for the producer host.

In spite of the great number of proteins that have been expressed in *P. pastoris* using the *AOX1* promoter (Cereghino and Cregg, 2000, FEMS Microbiology Reviews 24: 45-66), it is of particular interest to have another promoter regions that respond to the presence or absence of carbon sources other than methanol. The above mentioned have particular significance during scale up of fermentations where large volumes of methanol are used.

As was mentioned previously, a further object of this invention is to develop an expression system in which the transcription of the recombinant gene depend of the carbon source present in the culture. In yeast the expression of the structural *ICL* gene is repressed if the carbon source present in the medium is glucose, and is induced in absence of glucose or in presence of ethanol. Due to this characteristic, could be attractive the use of the promoter region of this gene in order to lead the expression of a foreign proteins. In this sense, a novel expression system has been developed in *S. cerevisiae* using the promoter region of the structural *ICL* gene of *Candida tropicalis* (T. Kanai et al., 1996, Appl. Microbiol Biotechnol 44: 759-765).

In accordance with the present invention, a DNA fragment has been isolated corresponding to the 5' regulatory region of the *ICL* gene from *P. pastoris*. Another aspect of this invention is to provide a 3' transcriptional termination sequence of the *ICL* gene of *P. pastoris*.

The last aspect of this invention is the construction of a vector that contains a DNA fragment derived from the *ICL* gene of *P. pastoris*, fused to the dextranase structural encoding gene (*dexA*) of *Penicillium minioluteun*. This DNA fragment, corresponding to the promoter region of *ICL* gene can regulate the expression of *dexA* gene in the host strain.

The *ICL* 5' regulatory region and the *ICL* 3' transcriptional termination sequence can be recovered from *P. pastoris* cultures such as BKM90 *P. pastoris* strain (BKM, Moscow) by a method described in the examples bellow. The general methodology employed for the isolation of the gene are known by those of skill in the art and consisted in the isolation of a DNA probe corresponding to the encoding region of the gene.

This probe was obtained by the polymerase chain reaction. For that purpose, the alignment of the amino acid sequence available for the proteins from several species allowed the design of degenerated oligonucleotides (identified in the list of sequences such as Seq. 3 and Seq. 4, where W is A or T, R is A or G, Y is C or T, H is A, C or T and N are A, C, G or T).

Using these oligonucleotides as primers and *P. pastoris* genomic DNA as a template for the PCR reaction, one fragment of 1.2 kb was obtained. Total genomic DNA was digested with several enzymes and hybridized with the labelled 1.2 kb DNA fragment. The hybridization was carried out according to Sambrock et al. (1989). Taking into account the results from the Southern blot experiment, a genomic DNA library from *P. pastoris* was constructed according to Sambrock et al. (1989).

The DNA sequence of the fragment isolated from the genomic DNA library that contain the entire gene as well as the regulatory regions, was determined by the dideoxy chain termination method (F. Sanger et al., 1977, P.N.A.S. 74: 5463-5467). The *ICL* 5' regulatory region is contained within the DNA fragment extending from the *Eco*R I site at -684 to about the *Ava* II site near the beginning of the structural gene at +7 position.

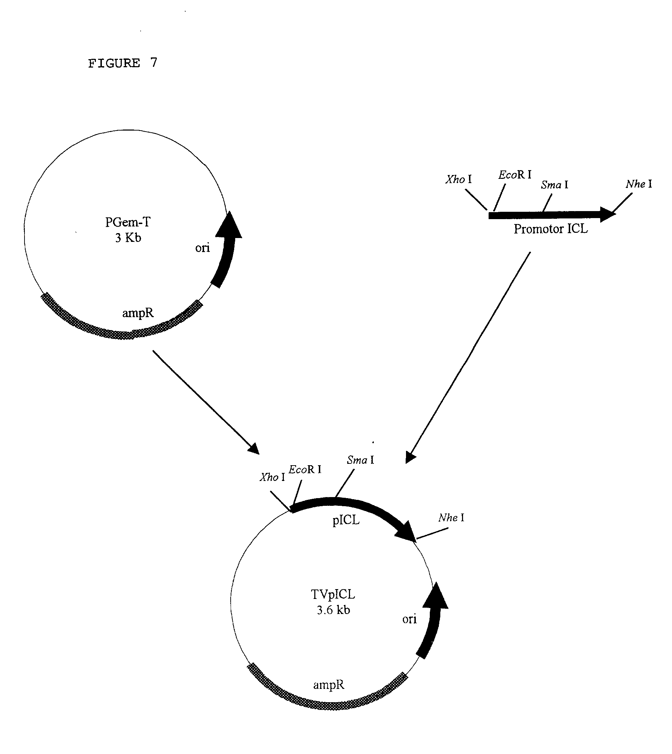

Specifically, the *ICL* 5' regulatory region corresponding to a fragment of 684 bp, was cloned into the pGEM® -T vector (Promega, USA) to give the plasmid TvpICL (figure 7). This plasmid was introduced into the *E. coli* TOP10F'strain [F'{tet^{r}} *mcr*A Δ(*mrr-hsd*RMS-*mcr*BC) φ80Δ*lac*-ΔM15 *Δlac*X74 *deo*R *rec*A1 *ara*D139 Δ(ara,leu)7697 galU galK λ⁻ rpsL endA1 nupG] (Invitrogen, San Diego, California) which was named TPIC-1.

In order to use the *ICL* 5' regulatory region disclosed herein, the 684 pb fragment can each be operably linked to heterologous DNA sequences encoding at least one polypeptide. For the purpose of this specification heterologous DNA sequences are combinations of DNA sequences which do not naturally occur in association with said regulatory regions. Suitable heterologous DNA sequence encoding at least one polypeptide which could be operably linked with the *ICL* 5' regulatory region include but are not limited, to dextranase encoding gene from *P. minioluteum*. The heterologous DNA sequence used with the present invention should contains a 5' ATG start codon, a 3' stop codon.

The combination of the *ICL* 5'regulatory region operably linked to a heterologous DNA sequence may be inserted in a suitable vector. Numerous useful vectors have been described for yeast transformation and are known to those skilled in the art. They should contains necessary elements which render the vector capable of growth amplification and rapid propagation in bacteria or yeast.

Suitable host cells for the use of the *ICL* 5'regulatory region include yeast such as *Saccharomyces, Hansenula, Pichia* and *Candida* and particularly *P. pastoris*. The introduction of the promoter region into the host strains by a compatible vector can be accomplished by suitable transformation techniques known to those skilled in the art.

The *ICL* 5'regulatory region when operably linked to a heterologous DNA sequence which has been transformed into a suitable host will express the heterologous DNA sequence in response to the carbon source present in the medium. During the growth in glucose, this carbon source repress the transcription of gene and this can take place when glucose is depleted from the medium or when ethanol concentration is about 3% by weight per volume of feed.

The *ICL* 3' transcription termination sequence of the *ICL* gene is believed to terminate mRNA transcription or stabilize mRNA when linked 3'to a DNA sequence which encode a polypeptide. This 3' transcriptional termination region was further characterized by the partial nucleotide sequence and corresponded to the fragment comprising between nucleotide 2337 to the nucleotide 2697. The *ICL* 3' transcriptional termination sequence may be operably linked to a heterologous DNA sequences which codes for a polypeptide and utilized to terminate transcription of or stabilize mRNA in yeasts such as *Saccharomyces, Hansenula, Candida* and *Pichia* but it is particularly well suited for use in *P. pastoris*.

The following practical examples are provided to illustrate this invention.

### Microorganism Deposit

The TPIC-1 strain was deposited according to the Budapest Treatment on the BELGIAN COORDINATED COLLECTION OF MICREOORGANISM- BCCM™ LMBP-COLLECTION, Gent, Belgian with the following accession number: LMBP 4107, May 15, 2000.

### EXAMPLES OF REALIZATION

### Example 1. Regulation of ICL gene from Pichia pastoris.

The regulation of isocytrate lyase in strain of *P. pastoris* BKM90 was measured through out the determination of the enzymatic activity in different culture conditions, specifically in repression conditions (exponential phase) as well as in de-repression conditions (stationary phase).

Yeast cells were grown in 50 ml of YP medium, that contains 10 g of yeast extract, 20 g of peptone in 1 liter of water, or in YP medium in which 20 g of glucose (YPD medium) or 25 ml of ethanol (YPE medium) were added. An equivalent of 100 mg of cells were collected at O. D.₆₀₀ₙₘ of about 1 (wet weight) and were washed twice with 20 ml of distilled sterile water and frozen at -20°C for it further analysis (culture in exponential phase). The remained culture was incubated until an O. D.₆₀₀ₙₘ of about 10, and an equivalent of 100 mg of cells (wet weight) were colleted and processed in the same way that we mentioned above (culture in stationary phase).

For cell-free protein extract preparation, each sample was defrosted on ice and washed once in distilled water, once in lysis buffer (50 mM Tris-HCl pH= 7.4, 1 mM DTT) and resuspended in 500 µl lysis buffer. 1g of 0.45 mm glass beads were added to a 500 µl aliquot of sample, and the mixture was vortexed three times for 60 seconds each with 1 minute intervals on ice. The cell slurry was removed from the glass beads by centrifugation and the supernatant was transferred to a another microfuge tube for assaying. The amount of total protein in each extract was determined by the Lowry assay method. BSA served as the protein standard (Lowry et al., 1951, J.Biol.Chem. 193: 265-269).

The ICL activity was determined using 10 µl of cell-free protein extract, which was added to 500 µl of the reaction mixture formed by the reaction buffer (1M KH₂PO₄ pH=7; 1 M MgCl₂, 2 M KCl) plus 20 mM reduced glutatione and 60 mM hydrochlorate of phenilhidrazine. The reaction was initiated by the addition of 150 µl of 0.4 M isocitrate and the absorbance at 324ₙₘ was then read (Dixon and Kornberg, 1959, Biochem J.72: 3P). One unit was defined as the amount of enzyme that catalyze the formation of one µmol of glyoxilic acid in a minute at 37°C and pH =7.

**Table 1.**

| Specific activity of isocitrate lyase measured in cell extracts from cells of *Pichia pastoris* grown in different carbon sources. E: exponential phase, S: stationary phase. | | |
|---|---|---|
| | Act. ICL (mU/mg)* | |
| Culture medium | E | S |
| YPD | <1 | 48 |
| YPE | 89 | 66 |
| YP | 39 | 21 |

| | | |
|---|---|---|
| *Enzyme activity was expressed as nmoles product/mg of protein/minute and represent an average of determinations from three independent cultures. | | |

In culture grown in glucose as the only carbon source, ICL activity in exponential phase of growth was not detected. In these conditions the expression of the enzyme is not required. However in stationary phase the enzyme is necessary to metabolize the ethanol produced during exponential phase. In cells grown in YP medium or YP medium suplemented with ethanol, the enzyme was detected.

Similar evidences have been reported for the expression of *lacZ* gene that encodes β-galactosidase, under control of *ICLI* promoter in *S. cerevisiae*. Using an *ICL-lacZ* fusion integrated at the *ICL1* locus, more than 200-fold induction of β-galactosidase activity was observed after growth on etanol when compared with glucose-repressed conditions (Schöler and Schüller, 1993, Curr. Genet.23: 375-381).

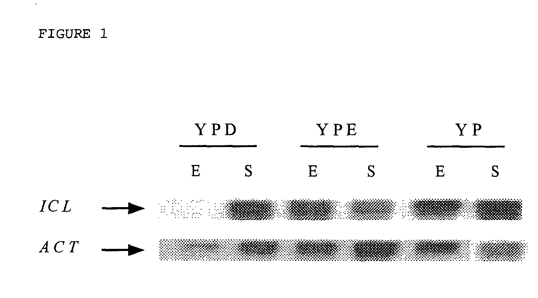

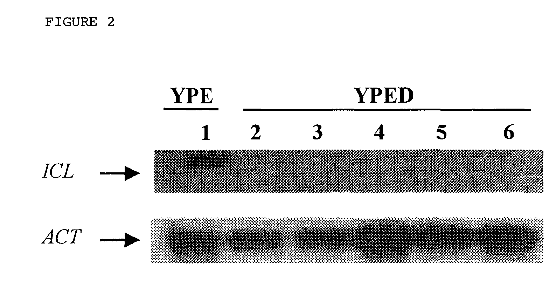

To knowing if the expression of the enzyme is regulated at the transcriptional level, northern blot hybridization (Sambrook et al., 1989) with RNA from cells grown in the same conditions described above was performed. Figure 1 showed the regulatory effect of *ICL* on the transcript level. The comparison with the constitutively expressed actin RNA shows that the specific *ICL* mRNA may be synthesized at a considerable level under appropriate conditions. This conditions referred to an absents of glucose in the medium. However, glucose not only repressed the expression of the *ICL* mRNA but also caused a reduction of the *ICL* mRNA after the shift of ethanol grown cells to a medium with glucose (figure 2). The latter mechanism has been reported for other genes subject to glucose repression.

### EXAMPLE 2. Isolation of Pichia pastoris ICL gene.

Isocitrate lyase has been described in some eukaryotic and prokaryotic organism and several genes encoding them have been isolated from different sources. Alignment of the amino acids sequences available for the proteins from *S. cerevisiae, Y*. *Lipolytica*, and *C. tropicalis* allowed the design of degenerated oligonucleotides corresponding to conserved N-terminal (Seq. 3) and C-terminal (Seq. 4) regions of protein.

Using these oligonucleotides as primers and *P. pastoris* genomic DNA as a template for the PCR reaction, three fragments of 0.5, 0.7 and 1.2 k respectively, were obtained. The technique of PCR was carried out according to procedure described in literature (Saiki et al, 1988, Science 239: 487-491).The 1.2 kb fragment has the expected size according to the *ICL* gene sequence from *S. cerevisiae* (Fernández et al., 1992, Eur. J. Biochem. 204: 983-990).

This fragment was ligated into pMOSBlue (Amersham) and the resulting plasmid was named TvICL. Sequences from both ends showed a high homology (70%) with those of other *ICL* genes.

The 1.2 kb *Nco* I/ *Eco*R I fragment from TvICL plasmids was isolated from a low gelling temperature agarose (SIGMA) and it was labelled with α-ATP³² using the Klenow DNA polymerase fragment (Sambrok et al., 1989). 10 µg of total genomic DNA was digested with the following enzymes: *Bam*H I, *Bgl* II, *Eco*R I and *Hind* III and hybridized with the labelled 1.2 kb fragment. In all cases one band of 9, 6, 4 and 1.9 kb respectively, was observed. This probe was also used to screen a library of *P. pastoris* genomic DNA. This library was constructed by inserting the fragments (range in size between 3.5 and 5 kb) from the *Eco*R I digestion into the pBluescript vector (Amersham).

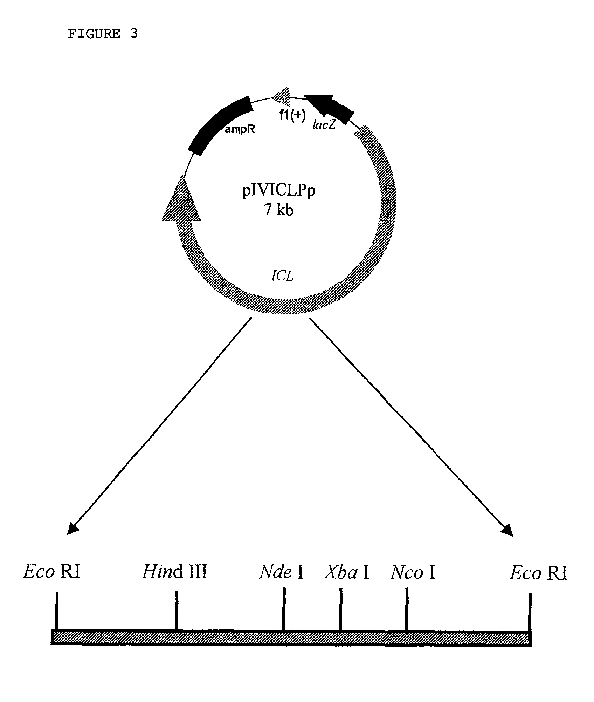

The library was screened by transforming competent MC1061 bacterial cells (Sambrook *et a*l., 1989), plating this cells on LB and 50 mg/ml ampicillin plates, and performing filter lifts as described in Grunstein and Hogness, 1975, PNAS. 72:3961. Individual positive colonies were examined by restriction analysis digestion of DNA prepared by standard miniprep procedure (Sambrook *et al.*, 1989) and the isolated plasmid was designated as pIVICLPp1-5. The restriction map of this plasmid is shown in figure 3.

### EXAMPLE 3. Secuenciation of Pichia pastoris ICL gene

DNA sequencing of the *ICL* gene was performed by the dideoxy chain termination method (F. Sanger et al., 1977, P.N.A.S. 74: 5463-5467). A 2732 pb fragment from the pIVICLPp plasmid was sequenced using universal oligonucleotides from M13mp/pUC series as well as specific oligonucleotides derived of the obtained sequence. This fragment contains an open reading frame of 1653 pb (551 codons). This gene encode for a protein with a calculated molecular weight of 60610 Da. The 5' uptream non-coding region (Seq.1) show a possible TATA box (TATAA) starting at position -96 regarding to ATG.

Comparison of the amino acids sequence deduced from the sequence of *P. pastoris ICL* gene with the amino acid sequence of others *ICL* genes available in the data bases showed a 70% of identity with the gene of *C. tropicalis*, a 63% with *Y. lipolytica* and *S. cerevisiae* gene and a 58% with *Neurospora crassa* gene.

### EXAMPLE 4. Functional analysis of the isolated DNA fragment

Functional analysis of the cloned gene was carried out according to the following examples:

### (1) Expression of the ICL gene in a S. cerevisiae icl⁻.

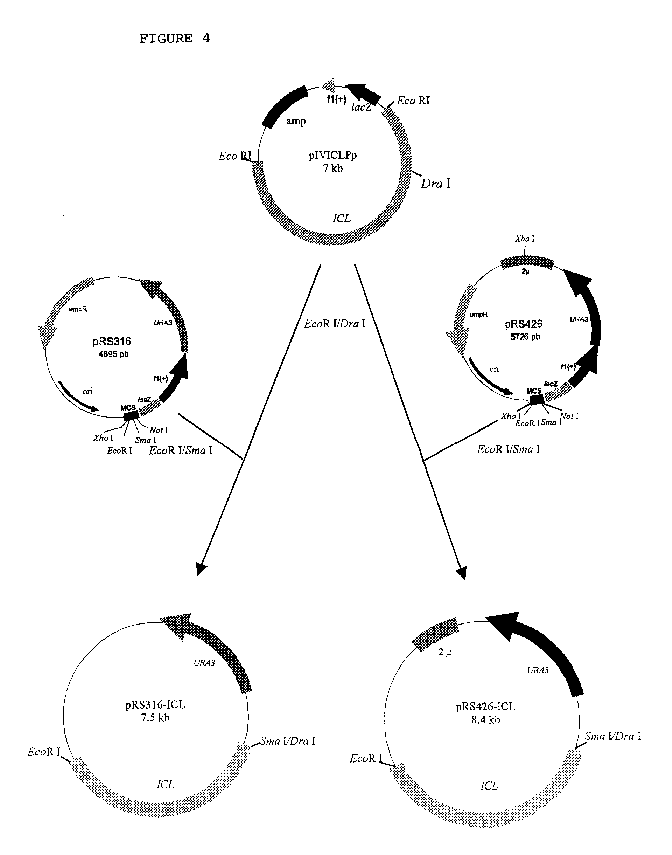

The following constructions were done to express the *P. pastoris ICL* gene under its 5' non-coding region in *S. cerevisiae.* The vectors pRS316 (Sikorski and Hieter, 1989, Genetics 122: 19-27) and pRS426 (Christianson et al., 1992, Gene 110: 119-122) were digested with the *Eco*R I and *Sma* I restriction enzymes and ligated with the 2.5 kb *Eco*R I-*Dra* I fragment extracted from the plasmid pIVICLPp to give rise to plasmids pRS316-ICL and pRS426-ICL (figure 4).

These were introduced in a strain of *S. cerevisiae* deficient in the *ICL* structural gene encoding for isocitrate lyase (strain FMY401). The transforming strain was able to grow in minimal medium plates with ethanol as the only carbon source showed that the cloned gene is functional in an heterologous host.

### (2) Disruption of the ICL gene and resulting phenotype (icl⁻)

The disruption of the isolated fragment in the present invention was carried out using the *HIS3* gene of *S. cerevisiae*, which is expressed efficiently in *P. pastoris* under its own promoter region (Yong et al., 1991, Biotecnol Aplic. 9: 55-61). The use of the *HIS3* gene of *S. cerevisiae* reduce the selection of false positive clones during the transformation event, due to a reduction in the gene conversion frequency when this gene is used (García et al., 2000, Biotecnol Aplic. 17: 11-15).

In order to carry out the disruption, the DNA fragment present in the pIVICLPp plasmid, was digested with the *Eco*R I enzyme and blunt ended using the Klenow fragment and also was digested with *Dra* I enzyme and the 2.8 kb fragment was cloned into the vector pOV10 (Vincent and Gancedo, 1995, Curr. Genet 27: 387-389), that was previously digested with *Sma* I to give rise the plasmid denominated pOVICL.

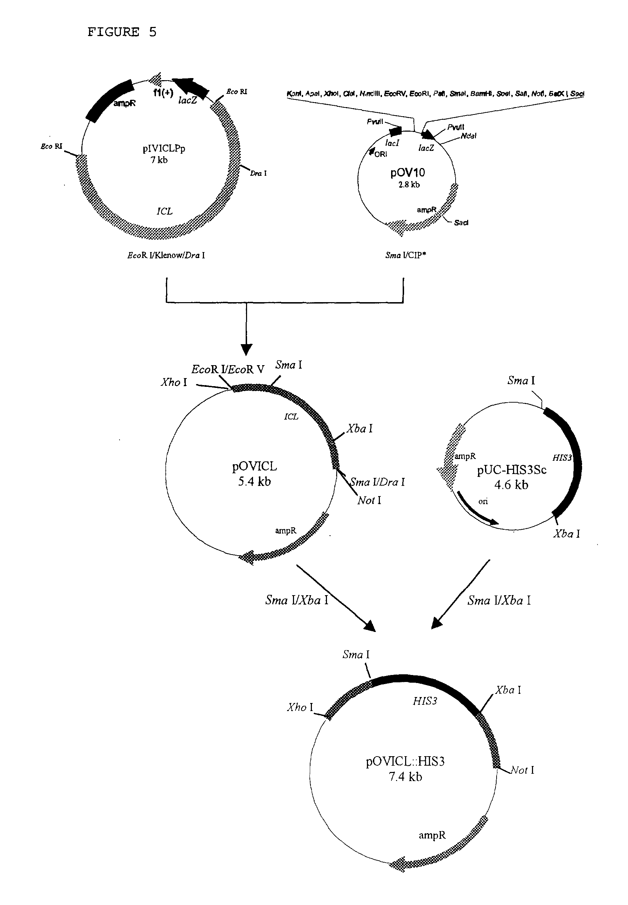

Finally, the 2 kb fragment that contains the *HIS3* gene of *S. cerevisiae* was obtained from the pUC-HIS3Sc plasmid, which was digested with *Sma* I and *Xba* I. The *HIS3* gene was them ligated with the pOVICL vector previously digested with *Sma* I and *Xba* I, giving the plasmid pOVICL:: HIS3 (figure 5).

In this plasmid the *HIS3* gene remains flanked in both sides with 400 bp from the *ICL* gene which are used in order to direct the site specific integration. This plasmid was digested with *Xho* I and *Not* I previous to the transformation. The fact that the transformed strain could not grow in minimal medium with ethanol as the only carbon source, together with the result of the experiment described above, indicate that in *P. pastoris* there is only one gene encoding isocitrate lyase. The isolated and sequenced fragment of the present invention correspond with the *ICL* structural gene of *P. pastoris*.

### EXAMPLE 5. Construction of the plasmid pPICL-DEX

For the construction of the expression vector pPICL-DEX, firstly the of 684 bp fragment that contains the 5' regulatory region of the *ICL* gene (Seq. 1) was isolated by polymerase chain reaction (PCR). The oligonucleotides IC-15 (Seq. 7) and IC-17 (Seq. 8) were chemically synthesized. Following the amplification, the fragment of interest was purified in 0.8 % agarose gel and cloned into the vector [pGem-T] (Promega, USA) giving rise to the TvpICL plasmid (figure 7).

For the construction of the TVDEX plasmid, the dextranase encoding gene from *Penicillium minioluteum* was isolated by PCR using the oligonucleótides IC-18 (Seq. 9) and IC-19 (Seq. 10). The product of the reaction was cloned into the pGem-T vector (Promega, USA) giving rise to the TVDEX plasmid.

To fuse the *ICL* 5' regulatory region with the dextranase encoding gene the TvpICL plasmid was digested later with the restriction enzymes *Xho* I and *Nhe* I and the 684 bp fragment was cloned into the vector TVDEX previously digested with the enzymes *Sal* I and *Nhe* I. The resulting plasmid was denominated pIV-2.

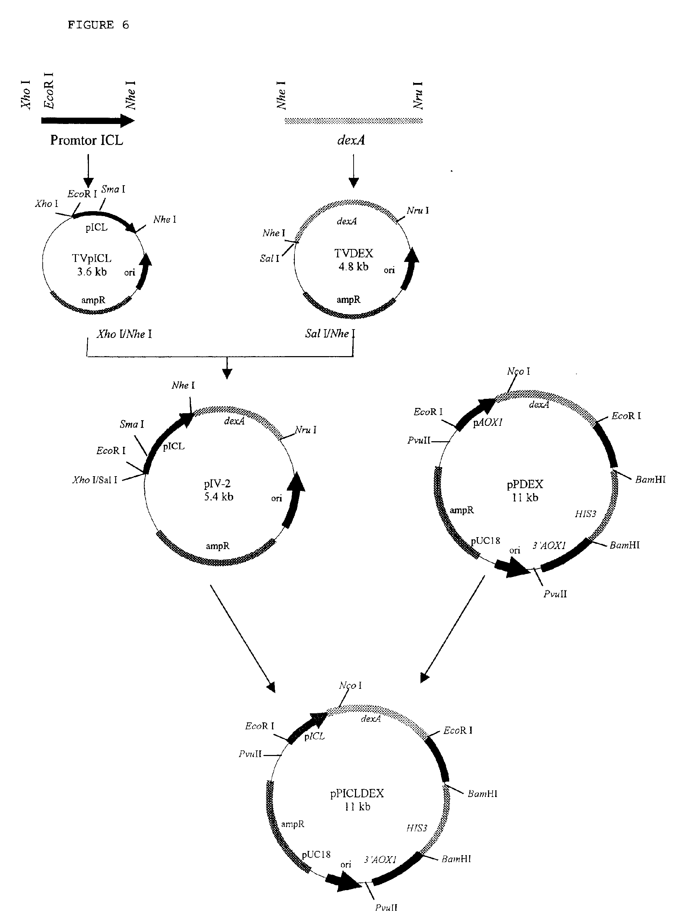

Finally, the plasmid pIV-2 was digested with the enzyme *Eco*R I and the product of the digestion, was cloned into the pPDEX vector (Roca et al., 1996, Yeast 12:1187-1200), previously digested with the *Eco*R I enzyme and treated with alkaline fosfatasa enzyme. The construction of the pPICL-DEX plasmid is show in figure 6.

### EXAMPLE 6. Expression of the dextranase structural gene using the promoter of the ICL gene.

The MP36 (*his3*^{*-*}) yeast strain of *P. pastoris* (Yong, 1992, Biotecnol Apl 9: 55-61) was transformed by electroporation method with the plasmid pPICL-DEX, essentially according to Becker and Guarente (1991, Methods Enzymol 194: 182-187). Previous to the transformation, the plasmid was digested with the *Sma* I enzyme in order to facilitate the integration in the genome.

The cells were pulse in 0.2 cm electroporation cuvettes at 1500 V, 25 µF, 400 Ω (BioRad Gene Pulser), 1 ml of cold 1 M Sorbitol was added immediately after the pulsing, and the His⁺ transformants were recovered in YNB plates with 2% of glucose. The plates were incubated to 28°C until the appearance of the transformants.

The functionality of the 5' fragment used was determined measuring the dextranase activity in cultures of 50 ml of YP medium supplemented with 2% of glucose or with 3% of ethanol as the only carbon sources. The activity was measured during the exponential phase of growth (DO₅₃₀ₙₘ of 1) and in stationary phase of growth (DO₅₃₀ₙₘ of 6). The culture supernatant was used in each case for the determination of the enzymatic activity.

### Dextranase activity assay:

The dextranase activity was determined according to Kosaric et. al. (Kosaric et al., 1973, Biotech Bioeng 15: 729-741), by means of the quantification of the reducing sugars formed by the action of the enzyme. The reducing sugars were determined colorimetrically by the dinitrosalicylic acid reagent method (DNSA) (Miller, 1959, Anal. Chem. 31: 426-428), proceeding in the following way: to 100 µl of samples were added 400 µl of 50 mM of sodium acetate tampon, pH 5.2 and 500 µl of 2.5% of dextrane T-2000 (Pharmacia). The reaction was incubated 10 min at 40°C and the enzyme was inactivated by heating 5 min at 100°C. One ml of DNSA was added to the reaction and was incubated 10 min at 100°C in order to develop the color. Finally the absorbance at 546 nm was determined against a blank in which the sample was substitute for 100 µl of acetate tampon.

In parallel, controls to determine the amount of reducing sugars present initially in the samples were prepared, in this case the dextrane was added after inactivation of the enzyme and the procedure was followed as for the determinations of enzymatic activity. One unit of activity was defined as the amount of enzyme that liberates 1 µmol of reducing sugar equivalents in 1 minute at pH 5 and was expressed in U/ml. The values of activities are shown in the table 2.

**Table 2.**

| Values of dextranase activities measured in the culture supernatant of the yeast *P. pastoris* grown in YP medium supplemented with glucose (YPD) or ethanol (YPE) as the only carbon sources. E: exponential phase of growth, S: stationary phase of growth. | | | |
|---|---|---|---|
| | | Dextranase Activity (U/ml) | |
| Strain | Carbon sources | E | S |
| MP36 (pPICL-DEX) | Glucose | <1 | 33 |
| | Ethanol | 9 | 18 |
| • Values are means of three independent experiments. | | | |

As is shown in the table 2, the strain transformed with the pPICL-DEX plasmid produced high levels of the dextranase enzyme in the cultures in which the ethanol was used as carbon source or in conditions of absence of glucose. This result confirmed that the expression of the structural gene codifying for the dextranase enzyme is regulated for the *ICL* promoter.

In this way was determined that the 5' regulatory region of the *ICL* gene of the yeast *P. pastoris* was able to direct the expression of the *dexA* gene of *P. minioluteum*, that means that this fragment could be used as an alternative promoter in the expression system of *P. pastoris* The expression of the *dexA* gene was regulated in response to the carbon source used being the expression of the protein controlled by the growth conditions used.

### DESCRIPTION OF THE FIGURES

Figure 1. Northern blot analysis of the RNA extracted from *P. pastoris* grown in several carbon sources. (E): RNA extracted of culture in exponential phase, (S): RNA extracted of culture in stationary phase. YPD, YPE and YP, rich mediums in which the glucose, ethanol or amino acids are used as carbon sources, respectively. The ICL and β-actin (*ACT*) genes were used as a probe, respectively.

Figure 2. Effect of the glucose on expression of *ICL* gene. RNA are hybridized with the *ICL* and β-actine genes. Line 1 corresponds to T= 0 and lines 2-6 represents the times starting from addition of glucose: 10, 20, 30, 60 120 min., respectively. The ICL and β-actin (*ACT*) genes were used as a probe, respectively.

Figure 3. Restriction map of *ICL* gene of *Pichia pastoris*.

Figure 4. Construction of pRS316-ICL and pRS426-ICL plasmids.

Figure 5. Construction of the pOVICL:: HIS3 plasmid.

Figure 6. Construction of the pPICL-DEX plasmid.

Figure 7. Construction of the TvpICL plasmid.

## Claims

1. Recombinants DNA fragments characterized because its contains the nucleotides sequences identified as Seq.1 and Seq .2, wherein said DNA fragments belong to the *ICL* gene isolated from *P. pastoris*, which are able to regulate the expression of an heterologous genes in yeasts, when these are operably linked to one of the said DNA fragment designated as Seq. 1 or Seq. 2 or with both.

2. A recombinant DNA fragment identified as Seq. 1 according to claim 1 wherein said DNA fragment has the nucleotide sequence described in the sequence list as Seq. 1, from about nucleotide 1 to 684, which compress the 5' regulatory region of the *ICL* gene isolated from *P. pastoris*.

3. A recombinant DNA fragment identified as Seq. 1 according to claims 1 and 2, characterized because is able to regulate the expression of an heterologous gene operably linked to the said DNA fragment, repressing the expression of the heterologous gene when glucose is present in concentrations of about 2%, or inducing the expression in absence of glucose or in presence of 3% of ethanol.

4. A recombinant DNA fragment identified as Seq. 2 according to claims 1, wherein said DNA fragment has the nucleotide sequence described in the sequence list as Seq. 2, from about nucleotide 1 to 360, which compress the 3' regulatory region of the *ICL* gene isolated from *P. pastoris*.

5. A recombinant DNA fragment obtained by recombinant or synthetic way and **characterized by** contains the nucleotide sequence of the fragments designated as Seq. 1 or Seq. 2, or part of these according to claims 1,2,3, and 4 and should include one or several regulatory elements necessary to the expression of an heterologous genes in an appropriate host cells.
